# EUROPEAN PATENT APPLICATION

(11) **EP 1 860 584 A2**
(43) Date of publication of application: **28.11.2007**
(21) Application number: 07252111.5
(22) Date of filing: 23.05.2007
(51) Int. Cl.: G06F 19/00

(54) **Systems and methods for providing individualized disease management**

(30) Priority: 24.05.2006 US 440341
(71) Applicant: Lifescan, Inc., Milpitas, CA 95035 (US)
(72) Inventor: Harmon, Kirk C., San Ramon CA 94582 (US)
(74) Representative: Tunstall, Christopher Stephen

(57) **Abstract**

Systems and methods of individualized disease management are provided and use patient-specific, physician-defined rules to assist a patient in the management of their disease. The set of physician-defined rules for a patient can be maintained within the patient's blood glucose metering system and activated when a lifestyle event or blood glucose result is expected or recorded. Pattern analysis can be performed in real-time to provide physician-generated suggestions to a patient to positively influence their behavior toward managing their disease.

## Description

### Technical Field

The present invention relates to systems and methods for managing health. More particularly, the present invention relates to systems and methods for providing individualized disease management to patients with a chronic disease.

### Background

Diabetes is a chronic disease that requires continued monitoring and controlling of health parameters such as blood glucose levels, medication, nutritional condition, as well as weight and exercise data. For patients with diabetes and their physicians, the amount of such information can be difficult to track and use effectively to make behavioral changes that positively influence management of their disease.

Further complicating matters is the fact that each patient brings a different personality to bear upon the treatment regime. That is, whereas some patients may respond quickly to reinforcement, whether positive or negative, so that very little reinforcement is required, others may require more repetition to cause a desired change. Effectiveness of positive versus negative reinforcement may also vary significantly among patients. For some patients, the necessity to interact with a medical or other device with any frequency, may be seen as a barrier that could negatively impact their ability to manage their disease, whereas others may actually enjoy such interaction and the sense of organization and control it can afford.

Each individual patient also brings different physical attributes and habits that influence their behavior and that can impact the effectiveness of a treatment plan. That is, while some diabetic patients may exhibit one or more of insulin resistance, aversion to dieting, and a relatively inactive lifestyle, others may respond to insulin, maintain a healthy diet and exercise regime but have a high level of stress. Typically, each patient may exhibit a combination of relatively positive and negative physical and behavioral attributes that may vary in occurrence over the course of treatment, as well as vary in significance in the context of each individual patients overall health and treatment progress.

Conventionally, several methods and systems to assist physicians and patients with the difficult task of diabetes management are available. Diabetes data management software, such as LifeScan's OneTouch™ Diabetes Management Software, for example, uploads results from a blood glucose metering system and stores this information in a database. This system, and others like it, may also attempt to integrate specific event information (i.e. tags, flags, and/or comments) or include additional lifestyle information (i.e. duration of exercise, nutritional information) that may impact a patient's blood glucose results. Subsequently, these systems can generate various reports when the physician or patient queries the database that may then be used to remind the patient, or alert a physician, of a past problem.

Although each of these types of methods and/or systems has provided invaluable assistance to physicians and patients alike in the complex task of disease management, each also may be limited in the assistance it can provide. That is, conventional methods and systems are not capable of responding to a patient's behavior or being customized in the information that is provided to a patient, much less in the information that is requested, or the frequency at which the information is provided or requested.

### Summary

In accordance with the present invention exception-based pattern analysis and reporting guidelines to process real-time data and provide physician-defined suggestions for disease management provide a useful alternative to conventional methods of disease management. Exception-based pattern analysis relies on a set of pre-set physician-defined rules that are patient specific to analyze in real-time all health parameters deemed necessary to track by the physician. Exception-based reporting provides real-time physician-defined suggestions based on the exceptions to the rules triggered by the exception-based pattern analysis module. Such a method may enhance both a patient's and a physician's ability to understand and actively influence patient compliance with disease state management.

The present invention uses patient-specific, physician-defined rules to assist a patient in the management of their disease. The set of physician-defined rules for a patient can be maintained within the patient's blood glucose metering system and activated when a lifestyle event or blood glucose result is expected or recorded. Pattern analysis can be performed in real-time to provide physician-generated suggestions to a patient to positively influence their behavior toward managing their disease. Moreover, a professional can download all stored data records from a patient user and generate a report detailing the results of a patient user for a period between office visits.

In accordance with an aspect of the present invention, a patient is assessed by a physician to define a set of rules for the management of the patient's disease. The assessment provides the physician with guidance as to setting of parameters of physician-defined rules. These parameters preferably include aspects of impact and significance. Impact may be a determination as to whether the measurement associated with the rule will have a positive or negative impact on the health of the patient. Significance may be how important the impact associated with the rule will be to the health of the patient. The significance may be viewed as a weighting of the impact to the rule. Further, the significance and impact may be viewed as rule parameters, among others that may be used to trigger reporting activities. Based on the assessment, the physician can also set parameters associated with rules that trigger a report to the patient, physician, or both. These parameters may be based on the physician's assessment of the patient and are preferably triggered based on the impact and the significance parameters of the rule being violated or complied with. If the reporting rule is triggered, a report or other output is preferably provided to the patient, physician, or both.

In another aspect of the present invention, a method of individualized disease management that customizes a pattern analysis rule and reporting trigger based on a disease management characteristic of a patient is provided. The method comprising the steps of defining at least one pattern analysis rule, determining at least one disease management characteristic of a patient, and customizing the at least one pattern analysis rule. The at least one pattern analysis rule comprises a rule parameter and a reporting trigger. The reporting trigger includes a rule parameter threshold. The method thus further includes a step of customizing the reporting trigger by adjusting the rule parameter threshold based on the at least one disease management characteristic of the patient.

In yet another aspect of the present invention, a system for individualized disease management is provided. The system comprises a data source, processor, memory, and program instructions. The program instructions comprise plural disease management parameters at least one of which is capable of being customized according to at least one disease management characteristic of a patient, obtain exception-based data from the data source, obtain at least one patient-specific physician-defined rule from the data source, and provide for the setting of at least one property for triggering the at least one patient-specific physician-defined rule.

### BRIEF DESCRIPTION OF THE DRAWINGS

These and other features, aspects, and advantages of the present invention will become better understood with regard to the following description, appended claims, and accompanying drawings where:
FIG. 1 is a schematic diagram illustrating an exemplary disease management system in accordance with the present invention;
FIG. 2 is a schematic view of an exemplary dialog window for interfacing with a user for providing settings for pattern analysis in a computer implemented method in accordance with the present invention;
FIG. 3 is a schematic view of an exemplary dialog window for interfacing with a user for providing settings for reporting rates in a computer implemented method in accordance with the present invention;
FIG. 4 is a schematic view of an exemplary output that may be sent to a professional user's output device in a computer implemented method in accordance with the present invention;
FIG. 5 is a schematic view of an exemplary output that may be sent to a patient user's output device in a computer implemented method in accordance with the present invention; and
FIG. 6 is a flowchart illustrating a sequence of steps in a method in accordance with the present invention.

### DETAILED DESCRIPTION

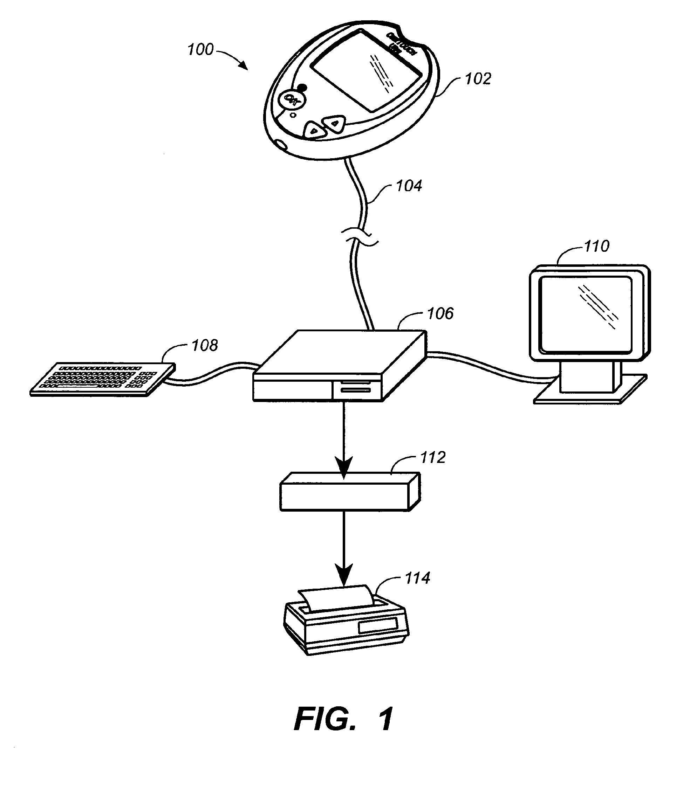

FIG. 1 illustrates an exemplary system 100 that implements a computer program 112 for exception-based pattern analysis and exception-based reporting in accordance with the present invention. System 100, as shown, includes a data source 102, a communications link 104, and a processing station 106 preferably connected to one or more data input devices 108, a visual display 110, and an output device 114. Examples of data source 102 include a blood glucose metering system and a continuous metering system for detecting glucose in blood or interstitial fluid such as described in U.S. Patent Application 10/432827, filed on 12/29/2003, which is fully incorporated herein by reference for all purposes. Other representative examples include metering systems for detecting analytes or indicators (e.g. cholesterol or HbA1c,) in any bodily fluid (e.g. blood, urine, interstitial fluid, etc). Generally, data source 102 may comprise any type of data input, including metering and measuring devices designed to test for physical characteristics. Data source 102 may further include input devices (e.g., buttons, keys, touch screens, on screen menus, user interfaces, etc.) to input lifestyle information such as, for example, quality and duration of exercise, weight data, type and quantity of diabetes medication, and general nutritional information.

As shown, data source 102 is connected to processing station 106 via communications link 104 and may comprise any known or future developed wired or wireless communications link. Examples of communications link 104 include a direct serial or USB cable, a TCP/IP or Ethernet based network connection and a wireless connection using protocols such as IEEE 802.11, InfraRed or Bluetooth. Alternatively, data source 102 can be connected directly to processing station 106 via an appropriate cable or the like.

Processing station 106 preferably includes a device to save and store information (e.g., a memory, a disk drive, or other removable storage device, a database, etc.) and a device to process data (e.g., a central processing unit or CPU) from data source 102 using algorithms within and desired software, such as within program 112. Examples of processing station 106 include a personal or networked computer, a personal digital assistant (PDA), a blood glucose metering system, and a mobile telephone. Examples of input devices 108 include, a keyboard, keypad, a mouse, a joystick, a stylet, as well as others which are usable with central processing unit devices. Examples of visual display 110 include, a display monitor for a personal or networked computer, and a Liquid Crystal Display (LCD) for a personal digital assistant (PDA), mobile telephone, and a blood glucose metering system. Alternatively, one or more lights, such as LED's, may be used on the device to communicate information by glowing and/or blinking. Examples of output devices 114 include, a printer, a fax machine, an email message, a text message, and a file that is stored to memory on processing station 106.

Processing station 106 further includes computer program or instructions 112 for providing exception-based pattern analysis in combination with exception-based reporting in accordance with the present invention. Exception-based pattern analysis can automatically notify the professional user (e.g. a physician or nurse practitioner, or anyone with an administrative function) or a patient user when any physician-defined metric or condition established in advanced is not being met or is being met. Exception-based reporting is designed to focus the attention of the professional user or a patient user on the exceptions to planned compliance to treatment and/or to praise appropriate behavior.

In general, data is collected by metering device 102 over a time period and typically includes plural samples. Exception based pattern analysis is preferably used to analyze the collected data and provide alerts, messages, or other information to a user (patient or professional). Pattern analysis preferably is used to identify general trends or patterns in data that is collected over time or from a number of samples. Exception based pattern analysis is generally a way in which to identify data which may fall outside acceptable data limits, and preferably exceptions to data limits of the disease management regimen. Pattern analysis rules are used to set acceptance levels for data in disease management. By looking for occurrences and/or average of occurrences that are outside acceptance limits, it may be apparent that the patient is not subscribing to the treatment regimen, or that there are other problems in the way that the disease is being treated or managed.

In a system in which the disease management can be customized to a patient, it is desirable that characteristics of a patient and their disease be assessed by a physician or other medical personnel such that the impact and significance associated with each of the pattern analysis rules is customized to the particular patient. For example, for glucose management, a rule might be set to track the number of times a glucose measurement or an average of all measurements is outside of a range, such as indirectly hypoglycemic or hyperglycemic conditions such a rule may be "report when averages for last 14 days are 20% below/over target or above upper target" where the impact is set to negative and the significance is set to the middle between low and high. Thus, every time the average over the last 14 days is outside the predetermined range, the rule is considered violated and, an exception is triggered. Once the exception is triggered, program 112 is configured to determine, based on the impact and significance parameters and the settings for reporting, whether a report, alert, or other indicator should be provided to a patient and/or professional user. The impact level and significance parameters and the settings for reporting are typically set by the professional user as detailed and explained below.

Computer program 112 preferably controls processing station 106 to perform many steps. Computer program 112 preferably utilizes standard user interfaces (e.g. menus and dialogs) to permit a user to access its functions. Computer program 112 may be written in any computer language, such as, for example, structured query language (SQL), Visual Basic, C++, as a matter of design choice and may be stored on any computer-readable memory device such as a hard drive coupled with a computer processing unit such as processing station 106.

Computer program 112 preferably includes both an exception-based pattern analysis unit and an exception-based reporting unit. Each of these units may be viewed as subroutines, or subprograms of computer program 112. Alternatively, these units can be separate programs which are called and initialized by computer program 112. Pattern analysis and reporting units may provide access to algorithms provided in software 112 or other separate software also provided in memory of device 106 for data sorting and analysis as well as expert system tools to help users control processes of computer program 112. Input data from data source 102 is incorporated into computer program 112 and the exception-based pattern analysis unit analyzes input data to determine if specific pattern criteria are met. The exception-based reporting unit then preferably generates reports for a patient user and an associated professional user (e.g., a physician, a diabetes educator, or a nurse). Such reports may be generated and viewed on any of a variety of devices, including device 102, processing station 106, display 110, and printer 114.

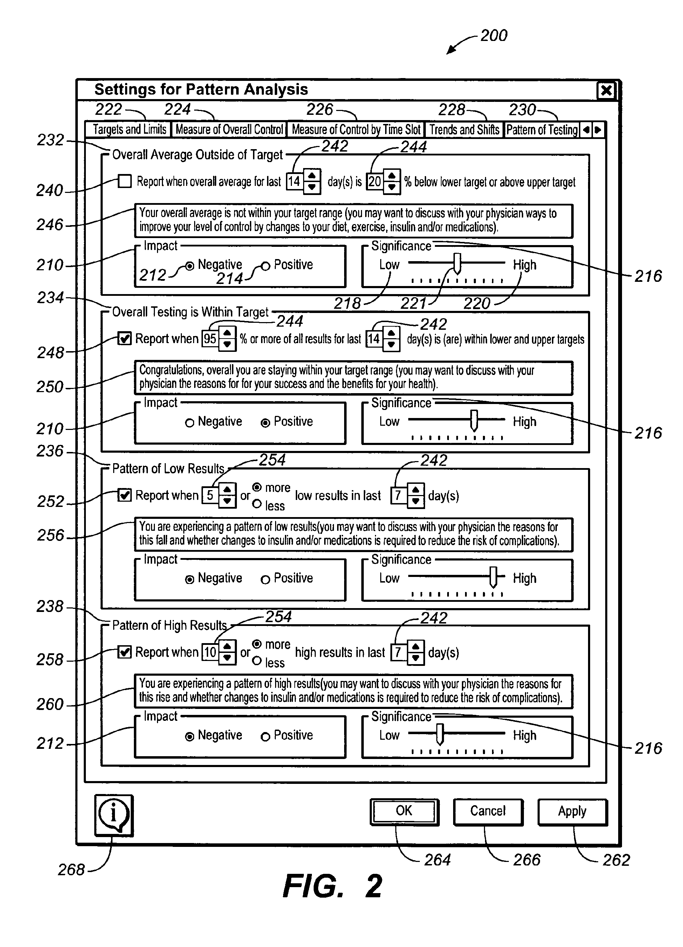

FIG. 2 illustrates an exemplary dialog window 200 that can be used for a professional user to set rules for pattern analysis, i.e. SETTINGS FOR PATTERN ANALYSIS. Window 200 may be displayed on visual display 110 or alternatively on another visual display that may be networked with processing station 106. Though listed in sequence, the selections which activate these rules may be selected at any time and can be changed interactively by a professional user at any time during the course of treatment of a patient user. In accordance with the present invention, the rules are preferably predefined in the software package by patient type such as a type 2 diabetic on a diet, a type 2 diabetic on oral medication, and gestational. Alternatively, the type of rules can preferably be changed/programmed by a professional user so that the software can be customized to a particular patient or physician's use. Implementation of changes to settings for pattern analysis typically occur at a communication link 104 between processing station 106 and data source 102. In other words, the settings are preferably downloaded to data source 102.

Optionally, the professional user may select in any order the physician-defined pattern analysis rules and provide settings therefore. The professional user may be a physician, nurse, other medical technician, data input personnel, any other administrative personnel, etc., having access to computer program 112. Also, a physician or other healthcare provider may redefine the physician-defined pattern analysis rules to customize to a particular disease or individual. The professional user may chose to select or not to select any of the pattern analysis rules, as well, depending upon the course of treatment for a patient user.

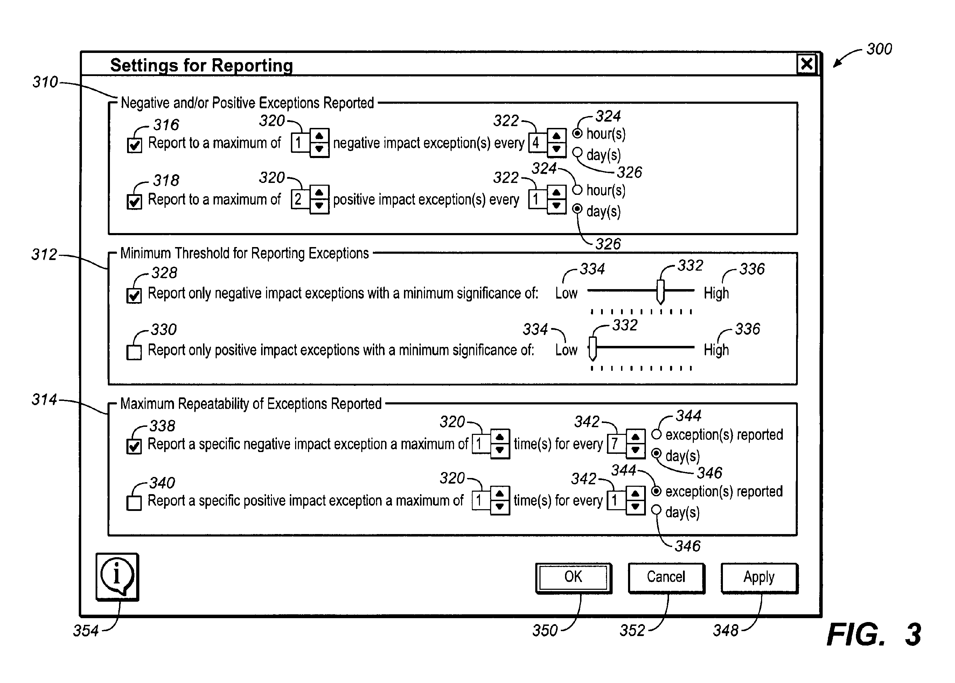

For each pattern analysis rule that a professional user selects, the professional user may set the limits for reporting the exceptions to and the properties of that pattern analysis rule. Limits are preferably set according to the rule and may include, for example, a duration (e.g., hours or days) and a percentage. For example, a physician may wish to set up the properties of a rule such that if the data source 102 measurement is on average at least 20% below the target for 2 days, then action (alarm, alert, report, etc.) is triggered. Properties that may be set up may also include an impact 210 and a significance 216. Impact may be a determination as to whether the measurement associated with the rule will have a positive or negative impact on the health of the patient. Significance may be how important the impact associated with the rule will be to the health of the patient. The significance may be viewed as a weighting of the impact to the rule. Further, the significance and impact may be viewed as rule parameters, among others that may be used to trigger reporting activities. The professional user may set impact 210 by clicking on a radio button preceding either Negative 212 or Positive 214 for each pattern analysis rule. The professional user may set significance 216 of a pattern analysis rule at either Low 218 or High 220 or some designation in between Low 218 or High 220 by moving a sliding bar 221 on a sliding scale. Thus, based upon a professional user's analysis or assessment of a patient, a professional user may decide whether violation (or compliance) of the rule has a negative or positive effect on a patient's treatment and how significant the violation (or compliance) with that rule is to the particular patient's treatment, including consideration of individual characteristics of the patient, (i.e., how the patient is likely to respond during disease management). Alternatively, other user interface setting options and mechanisms may be used, such as numerical choices, drop down menus, buttons, not limited to the radio buttons and slider bar illustrated in FIG. 2. Through a patient assessment by a physician or other medical personnel, the impact 210 and significance 216 for each pattern analysis rule are preferably set according to the patient characteristics determined during the assessment. For example, as in FIG. 2, hypoglycemia is viewed as being more significant by the physician than hyperglycemia. Thus, for a pattern of low results (i.e., low glucose readings over some duration), the significance 216 would be set at a High 220 level. Low results have a negative impact on the patient user's health. Therefore, a Negative 212 impact 210 would be selected in window 200. If a pattern analysis rule is triggered (based on the parameter set for the rule) and predetermined reporting properties of impact 210 and significance 216 are met or exceeded, a reporting exception to the rule may be triggered as described below with respect to Fig. 3. For example, when the pattern analysis rule and the reporting rule is triggered, one option is that the patient would receive a message about the exception. Typical messages to the patient user are provided below with respect to the descriptions of exemplary rules in Table 1.

Still referring to FIG. 2, window 200 for settings for pattern analysis rules, as shown, includes tabs for a TARGET AND LIMITS 222, a MEASURE OF OVERALL CONTROL 224, a MEASURE OF CONTROL BY TIME SLOT 226, a TRENDS AND SHIFTS 228, and a PATTERN OF TESTING 230. Although these are the tabs depicted in FIG 2, more or less tabs are contemplated.

In accordance with the present invention, TARGET AND LIMITS 222 tab preferably includes sections for setting one or more rules for an OVERALL AVERAGE OUTSIDE OF TARGET 232, an OVERALL TESTING IS WITHIN TARGET 234, a PATTERN OF LOW RESULTS 236, and a PATTERN OF HIGH RESULTS 238. For OVERALL AVERAGE OUTSIDE OF TARGET 232, a professional user may select limits X and Y for reporting exceptions by checking a box 240 preceding "Report when overall average for last X day(s) is Y% below lower target or above upper target" and by entering values for X and Y in boxes 242 and 244 (FIG. 2 depicts those values as being an exemplary 14 days and 20% respectively). For patients dealing with low blood sugar tracking, X may range from about 1 day to 14 days and Y may range from 0 percent to 100 percent, and more typically from about 10 percent to 20 percent. A professional user may select a number of days by toggling up and down arrows next to a day(s) box 242. A professional user may also select a percent by toggling up and down arrows next to a percent box 244. A professional user can also select impact 210 by clicking a radio button preceding either Negative 212 or Positive 214 (as discussed previously) and by sliding bar 221 for significance 216 (as discussed previously).

If an exception to this pattern analysis rule is triggered by collecting data over time, then a patient user may be prompted with the following exemplary statement: "Your overall average is not within your target range (you may want to discuss with your physician ways to improve your level of control by changes to your diet, insulin, and/or medication)," as shown in a reporting box 246. Interface window 200 provides customization of the trigger messages, e.g. as a text box allowing input thereto, or alternatively by a drop down or other access to predetermined message lists. A professional user may set the messages in reporting boxes 246, 250, 256 and 260 to be customized to a particular patient and to a particular disease. Computer program 112 may determine if the criteria for an exception to this pattern analysis rule are met and then may determine if the criteria for reporting this exception to a patient user are met. As an alternative to conventional systems, the present invention advantageously provides customization of the rules by the use of the impact and significance parameters determined via the physician's assessment of the patient's characteristics and the disease. The significance parameter is used in such a way that only rule violations that have at least a specified minimum significance are aggregated until a time is reached when the reporting criteria are met. Once the reporting criteria are met, the message is provided to the patient or professional user in order to affect behavior of the patient or for use by the physician as a way to better manage the patient's disease treatment.

For OVERALL TESTING IS WITHIN TARGET 234, a professional user may select limits Y and X for reporting exceptions by checking a box 248 preceding "Report when Y% or more of all results for the last X day(s) is (are) within lower and upper targets" and by entering values for Y and X. Y may range from about 0 percent to 100 percent and X may range from about 0 days to 21 days. A professional user may also select a percent by toggling up and down arrows adjacent to percent box 244. A professional user also may select a number of days by toggling up and down arrows adjacent to days box 242. A professional user can also select impact 210 by clicking a radio button preceding either Negative 212 or Positive 214 (as discussed previously) and by sliding bar 221 for significance 216 (as discussed previously). If an exception to this pattern analysis rule is triggered, then a patient user may be prompted with the following exemplary statement: "Congratulations, overall you are staying within your target range (you may want to discuss with your physicians the reasons for your success and the benefits to your health)" as shown in a reporting box 250. In this case, the message is positive which, when provided to a patient user may provide positive reinforcement to the patient user in order to reinforce the patient's good management of the patient's disease. Computer program 112 preferably determines if the criteria for an exception to this pattern analysis rule are met and may determine if the criteria for reporting this exception to a patient user are met, as described in more detail below.

For PATTERN OF LOW RESULTS 236, a professional user may select limits Z and X for reporting exceptions by checking a box 252 preceding a selection for "Report when Z or more (or less) low results in last X day(s)" and by entering values for Z and X in boxes 254 and 242 respectively. Z may range from about 0 to about 5 and X may range from about 0 days to about 21 days. A professional user may also select a number of low results by toggling up and down arrows adjacent to a numbers box 254. A professional user may also select a number of days by toggling up and down arrows adjacent to days box 242. A professional user can also select impact 210 by clicking a radio button preceding either Negative 212 or Positive 214 (as discussed previously) and by sliding bar 221 for significance 216 (as discussed previously). If an exception to this pattern analysis rule is triggered, then the patient user may be prompted with the following exemplary statement: "You are experiencing a pattern of low results (you may want to discuss with your physician the reasons for this fall and whether changes to insulin and/or medications is required to reduce the risk of complications)" as shown by a reporting box 256. In this case, the message is negative which, when provided to a patient user may provide negative reinforcement to the patient user in order to reinforce the patient's poor management of the patient's disease or to alert the patient that the treatment provided by a medical personnel is not effective and may need to be modified. Computer program 112 preferably determines if the criteria for an exception to this pattern analysis rule are met and may determine if the criteria for reporting this exception to a patient user are met, as described in more detail below.

For PATTERN OF HIGH RESULTS 238, a professional user may select limits Z and X for reporting exceptions by checking a box 258 preceding a selection for "Report when Z or more (or less) high results in last X day(s)," and by entering values for Z and X. Z may range from about 0 to about 10 and X may range from about 0 day to about 21 days. A professional user may also select a number of high results by toggling up and down arrows adjacent to numbers box 254. A professional user may also select a number of days by toggling up and down arrows adjacent to days box 242. A professional user can then select impact 210 by clicking a radio button preceding either Negative 212 or Positive 214 (as discussed previously) and by sliding bar 221 for significance 216 (as discussed previously). If an exception to this pattern analysis rule is triggered, then a patient user may be prompted with the following statement: "You are experiencing a pattern of high results (you may want to discuss with your physician the reasons for this fall and whether changes to insulin and/or medications is required to reduce the risk of complications)" as shown in a reporting box 260. In this case, the message is negative which, when provided to a patient user may provide negative reinforcement to the patient user in order to reinforce the patient's poor management of the patient's disease or to alert the patient that the treatment provided by a medical personnel is not effective and may need to be modified. Computer program 112 preferably determines if the criteria for an exception to this pattern analysis rule are met and may determine if the criteria for reporting this exception to a patient user are met, as described in more detail below.

It should be noted that for all of the pattern analysis rules 232, 234, 236 and 238, the system may not be limited to the rules shown and described. Any number of pattern analysis rules may be implemented in accordance with the present invention. The system shown and described provides a great deal of user flexibility by giving the users the ability to create nearly any type of rule.

Still referring to FIG. 2, to accept all of the appropriate pattern analysis settings for each patient, a professional user may click an APPLY button 262 or an OK button 264 and to cancel without accepting any changes to the pattern analysis setting options, a professional user may click a CANCEL button 266. When settings are changed, the metering device 102 needs to be updated with the new settings and may be done so at any later time including during the next communication between processing station 106 and device 102. If the setting changes are canceled, the device remains as-is. To obtain more information about any feature in this window a professional user may click on an information button 268.

As shown in FIG. 2, other tabs for setting rules may include a MEASURE OF OVERALL CONTROL 224, a MEASURE OF CONTROL BY TIME SLOT 226, a TRENDS AND SHIFTS 228, and a PATTERN OF TESTING 230. The format for the windows in each tab may be similar to the window for TARGETS AND LIMITS 222 in that each rule preferably includes a section for setting limits for reporting exceptions and sections for setting properties such as impact 210 and significance 216. Exemplary tab windows with rules and exemplary limit statements with limits for reporting exceptions are listed in Table I below. As described previously, the limits are preferably determined by a professional user and if an exception to a rule is triggered, a patient user is preferably prompted with an appropriate statement.

**TABLE I**

| TAB | RULE | LIMIT STATEMENT |
|---|---|---|
| Measure of Overall Control | Excessive Fluctuation of Results | Report when overall standard deviation for last _ day(s) is greater than mg/dL |
| | Over-Treating of Below Target Results | Report when _ below target result(s) followed by result above target within hour(s) |
| | Over-Treating of Above Target Results | Report when _ above target result(s) followed by result below target within hour(s) |
| Measure of Control by Time Slot | Time Slots with Excessive Fluctuation of Results | Report when standard deviation of any time slot for last _ day(s) is greater than mg/dL |
| | Time Slots with Results Outside of Target | Report when time slots with greater than _ % of results are outside of target for last day(s) |
| | Time Slots with Average Above Upper Target | Report when average for any time slot is _ % greater than the upper target for last day(s) |
| | Time Slots with Average Below Lower Target | Report when average for any time slot is _ % less than the lower target for last day(s) |
| Trends and Shifts | Recent Condition/Compliance Shift | Report when a change of _ % between most current _ days and the same number of days prior |
| | Weekdays vs. Weekend Trend | Report when _ % difference between weekday and weekend for last days |
| | Current vs. Previous Encounter Trend and/or Shift | Report when _ % more lows, more highs, less lows, and/or less highs between encounters |
| Pattern of Testing | Pattern of Skipped Testing | Report when _ consecutive hour(s) or more within the last _ day(s) without testing |
| | Insufficient Overall Frequency of Testing | Report when the average number of results per day in the last _ day(s) is less than |
| | Insufficient Testing by Time Slot | Report when the total number of results per time slot in the last _ day(s) is less than |
| | Repeated Testing During Lows to Improve Overall Average | Report when _ or more results within _ minute(s) are below lower target within last day(s) |

In addition to the rules listed in Table I and described previously, those skilled in the art will recognize that the limit statements can be provided as "positive" rules instead of "negative" rules. For example, the first limit statement in Table I could be written as "Report when overall standard deviation for last _ day(s) is ***less*** than_mg/dL. Rules may also be included for other measured values (e.g., HbAlc results for a diabetic patient) or to implement an intensive insulin therapy protocol for use by nurses at the point-of-care. Also, the impact setting may be characterized as a less than or greater than setting.

FIG. 3 illustrates an exemplary dialog window 300 for a professional user to set rates for reporting, i.e. SETTINGS FOR REPORTING. For a patient user, a professional user may identify the rate at which positive and negative exceptions to pattern analysis rules are reported to a patient user. Window 300 for SETTINGS FOR REPORTING preferably includes sections for setting rules for NEGATIVE AND/OR POSITIVE EXCEPTIONS REPORTED 310, MINIMUM THRESHOLD FOR REPORTING EXCEPTIONS 312, and MAXIMUM REPEATABILITY OF EXCEPTIONS REPORTED 314. For NEGATIVE AND/OR POSITIVE EXCEPTIONS REPORTED 310, a professional user selects by checking a box 316 preceding the selection for "Report to a maximum of Z negative impact exception(s) every D hour(s) or day(s)" and/or by checking a box 318 preceding the selection for "Report to a maximum of Z positive impact exception(s) every D hour(s) or day(s)" where Z may range from about 0 to about 99 and D may range from about 0 hours to about 24 hours or from about 0 days to about 21 days, for the glucose monitoring example provided. A professional user may set how many positive or negative exceptions are reported within the time period by toggling up and down arrows adjacent to a numbers box 320. A professional user may also set the rate at which either positive or negative exceptions are reported by toggling up and down arrows adjacent to a time period box 322 and by clicking a radio button 324 preceding hour(s) or by clicking a radio button 326 preceding day(s). By adjusting these settings, a professional user is able to set, based on the characteristics of the patient, how often and with what intensity the patient is alerted as to not staying within the treatment regimen or is provided with positive reinforcement for staying within the treatment regimen. If for example, a professional user has determined that the patient benefits from frequent reminders, then the settings may be adjusted such that the frequency of reminders is likely to be high. However, some patients may be annoyed by frequent reminders and alerts such that they will tend to ignore them. If this patient characteristic can be determined from a patient assessment, the settings may be configured in such a way as to provide, in most instances, less frequent reminders and/or alerts.

For MINIMUM THRESHOLD FOR REPORTING EXCEPTIONS 312, a professional user may activate the rule either by checking a box 328 preceding the selection for "Report only negative impact exceptions with a minimum significance of :" and/or by checking a box 330 preceding a selection for "Report only positive impact exceptions with a minimum significance of." In either case, a professional may select a significance 216 by dragging a tab 332 of a sliding scale toward either a Low 334 or a High 336 end of the scale. The significance setting here may be generally seen as a rule parameter threshold setting. Upon assessing the patient characteristics, a physician preferably determines what rules, when violated, may most significantly impact the patient's disease management. Therefore by changing the significance settings, a professional user is able to control which rules should be monitored to manage the patient's disease in the most significant way.

For MAXIMUM REPEATABILITY OF EXCEPTIONS REPORTED 314, a professional user may set either by checking a box 338 preceding a selection for "Report a specific negative impact exception a maximum of Z time(s) for every E exception(s) reported or X day(s)" and/or by checking a box 340 preceding a selection for "Report a specific positive impact exception a maximum of Z time(s) for every E exception(s) reported or X day(s)" where Z may range from about 1 to about 10, E may range from about 1 to 5, and X may range from about 1 day to about 21 days. A professional user may set a number of times a specific negative or positive impact exception is reported by toggling up and down arrows adjacent to numbers box 320. The professional user may set the rate at which a specific negative or positive impact is reported by toggling up and down arrows adjacent to a frequency box 342 and by clicking on a radio button 344 preceding "exceptions" or by clicking on a radio button 346 preceding "day(s)." To accept all of the appropriate reporting rates for all patients, the professional user clicks an APPLY button 348 or an OK button 350 and to cancel without accepting any changes to the reporting rates the professional user clicks a CANCEL button 352.

To obtain more information about any feature in this window a professional user may click on an information button 354. Although a number of specific settings and ranges of settings have been provided, the invention is not limited to those disclosed. Other settings and ranges of settings in accordance with the present invention may be used.

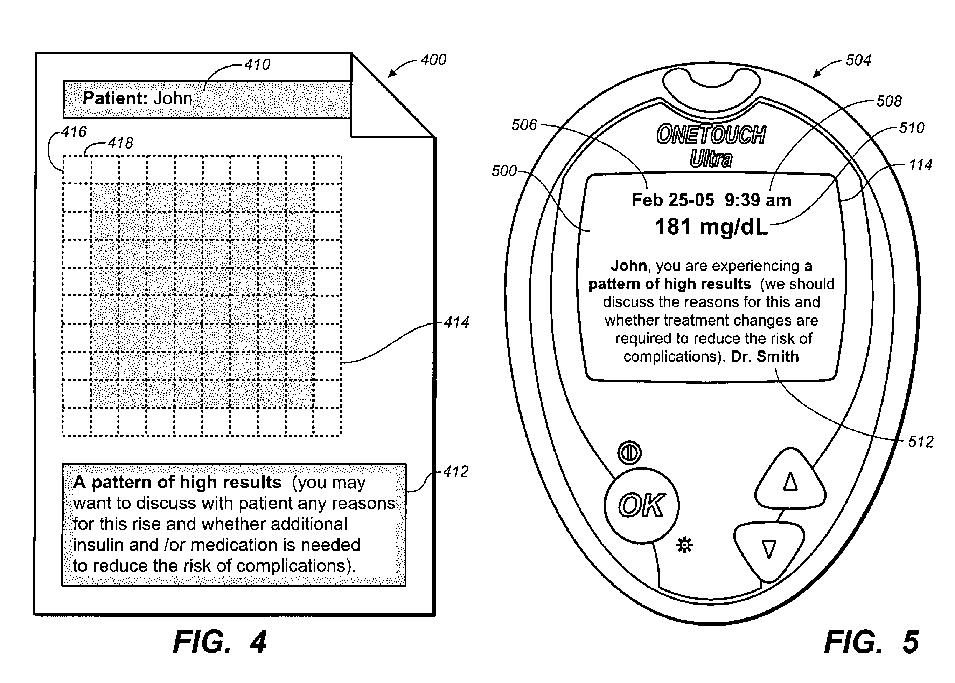

FIG. 4 illustrates an example of a professional report 400 that may be sent to a professional user's output device 114 by computer program 112. Professional report 400 preferably includes a title 410, a summary 412, and a data block 414. Title 410 preferably includes a means to identify a patient for which professional report 400 is generated. For exemplary purposes only, title 410 lists the patient's name, however, title 410 may include such information as metering system serial number, patient chart number, or other means to track patient information. Data block 414, as shown, includes multiple rows 416, each of which preferably includes data from one day of recording and multiple columns 418, each of which preferably includes data from one time period of recording. Examples of professional reports are further described in U.S. Provisional Patent Application 60/624804, filed on 11/2/04 and which is fully incorporated by reference herein for all purposes. Summary 412 preferably includes a list of statements based on physician-defined, pre-set criteria and generated by computer program 112 as described in more detail below.

FIG. 5 illustrates an example of a patient report 500 that may be sent to a patient user's output device 504 (e.g., a visual display) by computer program 112 in accordance with the present invention. Patient report 500 may also be sent to any output device, such as output device 114. Patient report 500 may include a location for a current date 506, a record time 508, a blood glucose result 510, and a patient summary 512. Patient summary 512 may include a statement based on physician-defined, pre-set criteria and generated by computer program 112.

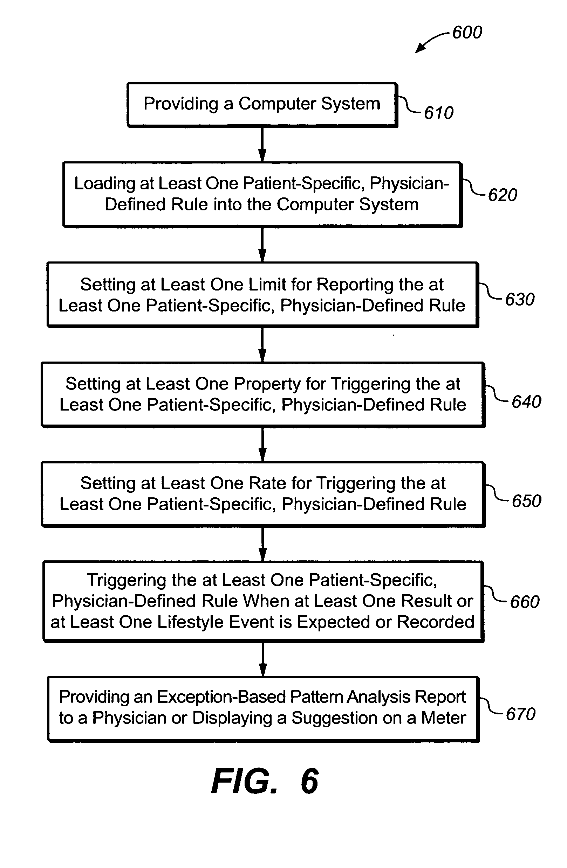

FIG. 6 is a flowchart illustrating an exemplary method 600 in accordance with the present invention. Method 600 preferably includes first providing a system 100 as described above with respect to FIGs. 1-5 and as set forth in step 610. The provided system preferably includes an input device, a processing device, and a reporting device for inputting, processing and reporting information associated with diabetes management. During process 600, individual lifestyle events and blood glucose results are integrated (e.g. uploaded or accessed) into computer program 112. Computer program 112 then analyzes the information based on a set of physician-defined rules for analysis and reports exceptions to physician-defined pattern analysis and reporting rules to a professional user and a patient user, as will be described below.

At least one patient-specific, physician-defined pattern analysis rule is defined and programmed into the system 100 and at least one limit for reporting the at least one patient-specific, physician-defined rule is set, as set forth in steps 620 and 630, respectively, and as illustrated in FIG. 2. A physician preferably selects one or more pattern analysis rules that are appropriate for the specific patient by, for example, checking a box 240 preceding the selection for "Report when overall average for last X day(s) is Y % below lower target or above upper target" where X may range from about 1 day to about 14 days and Y may range from about 10 percent to about 20 percent. The physician toggles up and down arrows adjacent to a days box 242 to set the appropriate number of days and adjacent to a percent box 244 to set the appropriate percent for this pattern analysis rule.

At least one property for triggering the at least one patient-specific, physician-defined rule is set by a physician as set forth in step 640 and as illustrated in FIG. 2. The physician preferably selects an impact 210 and a significance 218 for each pattern analysis rule established in step 620. To set impact 210, the physician clicks on a radio button preceding either Negative 212 or Positive 214 depending upon the needs of the specific patient user. To set significance 218, the physician slides a tab 221 on a sliding scale between Low 218 and High 220 depending on the needs of the specific patient user.

At least one rate for reporting the at least one patient-specific, physician-defined rule is preferably set by the physician as set forth by step 650 and as illustrated in FIG. 3. The physician sets the rate at which exceptions to reporting rules are triggered. To set a rate at which an exception patient-specific, physician-defined pattern analysis rule is reported to a patient user, the physician selects the rate by, for example, checking a box 316 preceding the selection for "Report to a maximum of Z negative impact exception(s) every D hours(s) or day(s)" where Z may range from about 0 to about 99 and D may range from about 0 hours to about 24 hours or range from about 0 days to about 21 days. The physician toggles up and down arrows adjacent to numbers box 320 to set the appropriate number and adjacent to time period box 322 to set the appropriate time period for reporting the exception to the pattern analysis rule established in step 620.

Next, at least one patient-specific, physician-defined rule is triggered when at least one result or at least one lifestyle event is expected or recorded as set forth by step 660. Computer program 112 preferably tracks exceptions and whether or not they are reported, which can be used for processing future pattern analysis exceptions. When input data is recorded or expected, computer program 112 analyzes pattern analysis rules and determines if an exception to any pattern analysis rule is triggered. If a pattern analysis rule is triggered then computer program 112 preferably determines if an exception to a reporting rate is triggered.

Computer program 112 then preferably provides an exception-based pattern analysis report to the physician or displays a suggestion to the patient on a metering system or to a physician or patient on an alternative output device as set forth by step 670. When computer program 112 determines an exception to a pattern analysis rule and a reporting rate is triggered, computer program 112 preferably generates a patient summary 512 that is displayed on a visual display 502 of a metering system 504 for a patient user. Computer program 112 also preferably generates the professional report 400 for the professional user when data from data source 102 is transferred to processing station 106 of professional user.

The software components as described above may comprise a stand alone computer program 112 or a computer module integrated into an existing computer program 112 such as, for example, the OneTouch™ Diabetes Management Software from LifeScan, Inc. In either configuration, computer program 112 preferably allows processing station 106 to accept data from data sources 102, to store incoming data, to process accepted and stored data using a main computer program 112 and a plurality of associated plug-ins in conjunction with a set of physician-defined control options, and to generate statements for both a patient user and the professional user to see.

Criteria of analysis and criteria of reporting are preferably stored in a non-volatile semiconductor storage element such as a ROM, flash memory, or a non-volatile storage device such as a hard disk or the like so that individual criterion can be added, deleted or modified as needed by the professional user. The function of each unit is realized by cooperative operation of hardware and computer program 112.

The present invention has now been described with reference to several embodiments thereof. The entire disclosure of any patent or patent application identified herein is hereby incorporated by reference. The foregoing detailed description and examples have been given for clarity of understanding only. No unnecessary limitations are to be understood therefrom. It will be apparent to those skilled in the art that many changes can be made in the embodiments described without departing from the scope of the invention. Thus, the scope of the present invention should not be limited to the structures described herein, but only by the structures described by the language of the claims and the equivalents of those structures.

## Claims

1. A method of individualized disease management, the method comprising the steps of:
defining an exception-based pattern analysis rule comprising a rule parameter and an exception-based reporting rule comprising a reporting trigger based on the rule parameter;
determining at least one disease management characteristic of a patient;
customizing the pattern analysis rule by adjusting the rule parameter based on the at least one disease management characteristic of the patient; and
customizing the reporting rule by adjusting the reporting trigger based on the at least one disease management characteristic of the patient.

2. The method of claim 1, wherein the step of determining at least one disease management characteristic comprises assessing the patient by a physician.

3. The method of claim 1, comprising defining a plurality of pattern analysis rules.

4. The method of claim 1, wherein the pattern analysis rule is at least partially defined by at least one of testing frequency, data input frequency, medication frequency, reporting frequency, and report content.

5. The method of claim 1, wherein the rule parameter of the exception-based pattern analysis rule comprises at least one of an impact parameter and a significance parameter.

6. The method of claim 1, wherein the at least one disease management characteristic of the patient comprises at least one of insulin receptivity, blood pressure, blood glucose, weight, cholesterol level, and glycosylated hemoglobin level.

7. The method of claim 1, wherein the at least one disease management characteristic comprises a psychological characteristic.

8. The method of claim 7, wherein the psychological characteristic comprises at least one of dietary habits, stress level, activity level, willingness to test, responsiveness to feedback, willingness to keep records, and ability to maintain a medication schedule.

9. A computer-implemented method for exception-based pattern analysis and exception-based reporting for individualized disease management, the method comprising the steps of:
defining at least one patient-specific, physician-defined rule;
setting at least one parameter that defines an exception to the at least one patient-specific, physician-defined rule;
setting at least trigger for reporting the exception to the at least one patient-specific, physician-defined rule; and
providing at least one physician-generated suggestion at a rate defined by a physician.

10. The method of claim 9, further comprising storing exception-based data and generating an exception-based report.

11. The method of claim 10, wherein the exception-based data is stored on and obtained from at least one of a glucose metering system, a web-based program, and a computer-based program.

12. The method of claim 9, wherein the at least one trigger for reporting the at least one patient-specific, physician-defined rule includes an impact of a trigger event and a significance of the impact of a trigger event to a patient.

13. The method of claim 12, wherein the impact of a trigger event is negative or positive.

14. The method of claim 12, wherein the significance of the impact of a trigger event is low to high.

15. The method of claim 9, wherein the rate defined by a physician includes at least one threshold for reporting a trigger event based on the impact of a trigger event, the significance of the impact of a trigger event, and the repeatability of reporting a trigger event.

16. A system for individualized disease management, the system comprising:
a data source, processor, and memory; and
program instructions comprising plural disease management parameters at least one of which is capable of being customized according to at least one disease management characteristic of a patient, obtain exception-based data from the data source, obtain at least one patient-specific physician-defined rule from the data source, and provide for the setting of at least one property for triggering the at least one patient-specific physician-defined rule.

17. The system of claim 16, wherein the data source comprises at least one of a glucose metering system, a personal data assistant, a mobile phone, a web-based program, and a computer-based program.

18. The system of claim 16, wherein the program instructions provide for the setting of at least one limit for reporting the at least one patient-specific, physician-defined rule.

19. The system of claim 16, wherein the program instructions trigger the at least one patient-specific physician-defined rule when at least one result is expected.

20. The system of claim 16, wherein the program instructions provide at least one physician-generated suggestion at a rate defined by a physician.
